# NEUE EUROPÄISCHE PATENTSCHRIFT

(11) **EP 1 628 626 B2**
(45) Veröffentlichungstag und Bekanntmachung des Hinweises auf die Entscheidung über den Einspruch: **04.12.2013**
(45) Hinweis auf die Patenterteilung: 11.08.2010
(21) Anmeldenummer: 03732324.3
(22) Anmeldetag: 07.05.2003
(51) Int. Cl.: A61K 8/02, A61K 8/37, A61K 8/46, A61K 8/60, A61Q 13/00, C11D 3/50, A61K 8/11, A61K 8/44, A61Q 5/02, A61Q 19/10, C11D 17/00, A61K 8/49, A61Q 5/00, A61Q 19/00

(54) **ZUSAMMENSETZUNGEN ZUR GEZIELTEN FREISETZUNG VON DUFTSTOFFEN UND AROMEN**
COMPOSITIONS FOR THE TARGETTED RELEASE OF FRAGRANCES AND AROMAS
COMPOSITIONS PERMETTANT UNE LIBERATION CIBLEE DE PARFUMS ET D'AROMES

(43) Veröffentlichungstag der Anmeldung: 01.03.2006
(73) Patentinhaber: OTC GmbH, 46047 Oberhausen (DE)
(72) Erfinder: DAHMS, Gerd, 47138 Duisburg (DE); JUNG, Andreas, 47198 Duisburg (DE); SEIDEL, Holger, 47055 Duisburg (DE)
(74) Vertreter: Michalski Hüttermann & Partner Patentanwälte
(86) Internationale Anmeldenummer: PCT/EP2003/004788
(87) Internationale Veröffentlichungsnummer: WO 2004/098555

(56) Entgegenhaltungen:
- EP-A- 0 466 235
- EP-A- 0 478 326
- EP-A- 1 243 323
- EP-A- 1 254 651
- WO-A-00/67728
- WO-A-00/72804
- WO-A-02/15862
- WO-A-02/26272
- WO-A-02/45575
- WO-A-02/50230
- WO-A-95/15143
- WO-A-99/43777
- WO-A2-02/43671
- WO-A2-02/43673
- DE-A- 10 152 898
- DE-A1- 10 016 155
- US-A- 4 226 890
- US-A- 5 788 975

## Beschreibung

Die Erfindung betrifft Zusammensetzungen zur gezielten Freisetzung von Duftstoffen und Aromen, speziell kosmetische, pharmazeutische, lebensmitteltechnologische oder waschmitteltechnologische Zusammensetzungen.

Viele kosmetische, pharmazeutische, lebensmitteltechnologische oder waschmitteltechnologische Zusammensetzungen enthalten Duftstoffe, worunter sowohl Duftöle als auch Aromastoffe verstanden werden sollen. Üblicherweise werden die Duftstoffe den Zusammensetzungen direkt beigemischt. Diese Vorgehensweise hat mehrere Nachteile. Zum einen kann bei der Anwendung die Freisetzung des Duftstoffs kaum gesteuert werden, so dass insbesondere eine retardierte Freisetzung nicht möglich ist. Zudem sind die Duftstoffe in der Regel nicht vor dem oxidativen Zerfall geschützt. Aus diesem Grunde müssen häufig größere Mengen an Duftstoffen in den Zusammensetzungen eingesetzt werden, um eine hinreichende Langzeitwirkung sowie eine ausreichende Wirkung nach längerer Lagerung zu erreichen.

Andererseits stellen Duftstoffe einen wichtigen kostenbestimmenden Bestandteil insbesondere von kosmetischen Zusammensetzungen dar. Wenn die Wirksamkeit der Duftstoffe beim Einsatz gesteigert werden könnte, wäre es möglich, mit geringeren Einsatzmengen auszukommen. In diesem Fall könnten kosmetische Zusammensetzungen kostengünstiger hergestellt werden.

Aufgabe der vorliegenden Erfindung ist die Bereitstellung von Zusammensetzungen zur gezielten Freisetzung von Duftstoffen und Aromen, insbesondere kosmetischen, pharmazeutischen, lebensmitteltechnologischen oder waschmittel-technologischen Zusammensetzungen, die die Verwendung geringer Einsatzmengen des Duftstoffs und/oder Aromas ermöglichen, eine gezielte, zum Beispiel retardierte oder kaskadierte Freisetzung des Duftstoffs und/oder Aromas erlauben und eine Oxidation des Duftstoffs und/oder Aromas verhindern.

Die Aufgabe wird gelöst gemäß Anspruch 1 durch eine Duftstoff- und/oder Aromen-Zusammensetzung zur gezielten Freisetzung von Duftstoffen und/oder Aromen in Form einer Solid-Lipid-Nanoparticle (SLN)-Dispersion, in der Nanopartikel auf Lipidbasis vorliegen, die durch eine Emulgatorenmonoschicht, eine oder mehrere Membranschichten oder andere Hilfsmittel stabilisiert sind. Die Duftstoffe/Aromen werden je nach Zusammensetzung in die festen Partikel und die die Partikel umhüllende Emulgatormembrane eingeschlossen. Die Duftstoffe/Aromen können auch im Bereich der hydrophoben Reste von Emulgatoren oder Tensiden vorliegen, die hydrophile und hydrophobe Reste enthalten.

Art und Dauer der Freisetzung hängen sowohl von der Verteilung des Duftstoffs/Aroma zwischen fester Lipidphase und der dazugehörigen Membranschicht, als auch des Schmelzpunktes der Lipidphase ab.

Hierin beschrieben wird eine vorliegend nicht beanspruchte Duftstoff- oder Aromen-Zusammensetzung zur gezielten Freisetzung von Duftstoffen oder Aromen, umfassend Tenside, die hydrophile und hydrophobe Reste aufweisen und in einer lyotropen lamellaren liquidkristallinen Phase vorliegen, wobei die Duftstoffe/Aromen je nach Löslichkeit oder strukturellem Aufbau in die Membrane/Lamellen selbst oder zwischen diesen eingeschlossen sind oder im Bereich der hydrophoben Reste der Tenside eingebracht sind.

Weiterhin beschrieben wird eine vorliegend nicht beanspruchte Duftstoff- und/oder Aromen-Zusammensetzung zur gezielten Freisetzung von Duftstoffen und/oder Aromen in Form einer PO- (Polyol-in-Öl) oder POW(Polyol-in-Öl-in-Wasser)-Emulsion. Der Duftstoff oder das Aroma sind dabei vollständig in der internen Polyolphase verkapselt, oder sie liegen im Bereich der hydrophoben Reste der Emulgatoren oder Tenside vor, wobei die Emulsion Emulgatoren oder Tenside mit hydrophilen und hydrophoben Resten umfasst.

Zudem wird beschrieben die vorliegend nicht beanspruchte Verwendung von Tensiden, die lyotrope lamellare liquidkristalline Phasen ausbilden, als Speicher zur gezielten Freisetzung von Duftstoffen und Aromen.

Die erfindungsgemäß beanspruchte Zusammensetzung kann dabei zur gezielten bzw. kontrollierten, zum Beispiel kaskadierten oder retardierten Freisetzung des Duftstoffs und/oder Aromas nach oder während der Applikation in kosmetischen, pharmazeutischen, lebensmitteltechnologischen oder waschmittel-technologischen Zusammensetzungen verwendet werden. Duftstoffe werden sowohl in leave-on-Produkten, wie z. B. Hautpflegeprodukten, als auch in rinse-off-produkten wie Shampoos, Waschmittel eingesetzt. Da Aromenstoffe, eingesetzt in Lebensmittel, nur eine begrenzte Verweildauer in der Mundhöhle haben, werden sie zu den rinse-off-Produkten gezählt.

Bei leave-on-Produkten möchte man ein möglichst lang anhaltendes Dufterlebnis erzielen. Dazu muss die Flüchtigkeit des Duftstoffes herabgesetzt werden. Des Weiteren erwartet man bei leave-on-Produkten die Bildung einer Duftpyramide, d. h. eine kaskadierte Freisetzung der in der Duftkomposition enthaltenen Duftstoffen.

Bei rinse-off produkten treten zumeist zwei Probleme auf. Zum einen wird der größte Teil des Duftstoffs mit der Waschflotte wieder von der Oberfläche, auf die er aufgetragen werden soll, abgetragen. Zum anderen verdunstet die verbleibende Restmenge relativ schnell. Die erfindungsgemäße Zusammensetzung gestattet durch oberflächenaffine Modifizierung von deren Zusammensetzung, eine stark verbesserte Haftung des Trägers und eine sehr lange Duftfreisetzung.

Zudem wird durch die erfindungsgemäße Zusammensetzung eine Oxidation der Duftstoffe bzw. Aromen vermieden.

Die Zusammensetzung liegt in Form einer Solid-Lipid-Nanoparticle (SLN)-Dispersion vor, in der eine lyotrope lamellare liquidkristalline Phase in Teilchen auf Lipidbasis vorliegen kann, aber nicht muss.

Beim Einsatz in SLN-Dispersionen kann das Duftöl/Aroma kontrolliert, zum Beispiel kaskadiert freigesetzt werden, so dass es zu einem langen Dufterlebnis kommt. Diese Ausführungsformen sind insbesondere für Leave On-Produkte vorteilhaft.

Pharmazeutische, kosmetische und/oder lebensmitteltechnologische Wirkstoffe werden häufig in festen Wirkstoffträgern, wie Gelatinekapseln, Cyclodextinen, Polymeren etc. verkapsuliert. Der Wirkstoffträger kann dabei an die jeweilige Anwendung angepasst werden und erlaubt eine geeignete Dosierung und Freisetzung des Wirkstoffs. In der Vergangenheit wurden feste Lipidnanopartikel, die auch als SLN (Solid-Lipid-Nanoparticles) bezeichnet werden, entwickelt Sie stellen ein alternatives Carriersystem zu Emulsionen und Liposomen dar. Die Nanopartikel können hydrophile oder hydrophobe pharmazeutische Wirkstoffe enthalten und können oral oder parenteral verabreicht werden. Üblicherweise werden dabei Nanopartikel mit einem mittleren Teilchendurchmesser im Bereich von 50 nm bis 1 µm eingesetzt. Als Matrixmaterial wird im Gegensatz zu den bekannten Emulsionen ein festes Lipid eingesetzt. Zur Gewährleistung einer hohen Bioakzeptanz und guter in-vivo-Abbaubarkeit werden überwiegend physiologisch verträgliche Lipide oder Lipide aus physiologischen Komponenten wie Glyceride aus körpereigenen Fettsäuren verwendet Bei der Herstellung werden üblicherweise Emulgatoren oder Tenside mitverwendet. Die Herstellung erfolgt in der Regel durch Hochdruckhomogenisierung. Dabei wird das als Matrix verwendete Lipid aufgeschmolzen, und ein Wirkstoff wird in der Schmelze gelöst oder dispergiert. Üblicherweise wird die wirkstoffhaltige Schmelze mit einer wässrigen Tensidlösung bei gleicher Temperatur unter Rühren dispergiert. Die so erhaltene Dispersion wird anschließend in einem Hochdruckhomogenisator, beispielsweise einem Kölben-Spalt-Homogenisator bei Drücken im Bereich von 200 bis 1500 bar im heißen Zustand homogenisiert. Es entsteht eine Emulsion, deren Lipidphase beim Erkalten zu festen Lipidnanopartikeln rekristallisiert.

Alternativ kann eine Kalthomogenisierung durchgeführt werden, bei der der Wirkstoff wiederum in eine geschmolzene Lipidphase eingebracht wird. Die erhaltene Mischphase wird danach abgekühlt, und der Feststoff wird auf eine Korngröße im Bereich von 50 bis 100 µm vermahlen. Die so erhaltenen Lipidteilchen werden anschließend in einer kalten Tensidlösung dispergiert, und die erhaltene Dispersion wird anschließend hochdruckhomogenisiert.

Die SLN-Dispersion ist erfindungsgemäß herstellbar durch
a) Vermischen des Duftstoffs/Aromas mit dem Wirkstoffträger auf Lipidbasis und mindestens einem Emulgator, der in Stufe b) zur Ausbildung einer lyotropen flüssigkristallinen Mischphase führt, bei einer Temperatur oberhalb des Schmelz- oder Erweichungspunktes des Wirkstoffträgers, zur Ausbildung einer Phase B
b) mechanisches Vermischen der Phase B mit einer wässrigen Phase oder Polyolphase A, die einen Emulgator enthalten kann, bei einer Temperatur oberhalb des Schmelz- oder Erweichungspunktes des Wirkstoffträgers, wobei das Gewichtsverhältnis von Phase B zu Phase A 1 : 5 bis 5 : 1 beträgt, ohne Hochdruckhomogenisierung, zur Ausbildung einer lyotropen flüssigkristallinen Mischphase,
c) Verdünnen der Mischphase mit einer wässrigen Phase oder Polyolphase, die einen Emulgator enthalten kann, bei einer Temperatur der wässrigen Phase oder Polyolphase, die unter dem Schmelz- oder Erweichungspunkt des Wirkstoffträgers liegt, zum Beispiel mindestens 15 °C darunter, unter Rühren und ohne Hochdruckhomogenisierung, auf eine gewünschte Endkonzentration der Dispersion wobei in der Duftstoff- und/oder Aromen-Zusammensetzung Lipide und Emulgatoren in einem Gewichtsverhältnis vom 50:1 bis 2:1 vorliegen.

Es wurde gefunden, dass wässrige Wirkstoffträger-Dispersionen, in der feste Wirkstoffträgerteilchen auf Lipidbasis mit einem mittleren Durchmesser im Bereich von 10 bis 1000 nm vorliegen, vorteilhaft hergestellt werden können, wenn eine Lipidschmelze mit einer auf die gleiche Temperatur aufgeheizten wässrigen Phase in dem bestimmten Gewichtsverhältnis von 1 : 5 bis 5 : 1 vermischt wird. Die Mischung kann dabei durch übliche mechanische Rührer erreicht werden, die die Rührleistung eines Haushaltsmischers (Mixers) (oder Haushaltsküchenrührers) aufweisen. Im Laborbetrieb war es beispielsweise möglich, mit einem Braun^{®}-Küchenmixer, der einen Mischkopf in Form eines zweiflügligen Propellers mit einem Gesamtdurchmesser von 50 mm aufweist, eine ausreichende Rührwirkung zu erreichen. Der Mischpropeller war von einem Schutzring mit einem Durchmesser von 63 mm umgeben. Die maximale Leistungsaufnahme des Küchenmixers betrug 350 W. Es handelte sich um das Modell MR 550, Type 4189.

Das mechanische Vermischen in Stufe b) und das Rühren in Stufe c) erfolgt vorzugsweise mit Rührern die eine Umfangsgeschwindigkeit im Bereich von 1 bis 20 m/s, besonders bevorzugt 1 bis 3 m/s aufweisen.

Vorzugsweise entspricht die Scherwirkung des Rührers dabei der Scherwirkung eines Haushaltsküchenrührers oder Mixers, wie er handelsüblich ist und vorstehend beschrieben wurde.

Das Gewichtsverhältnis von Phase B zu Phase A in Stufe b) beträgt vorzugsweise 1 : 2 bis 2:1, besonders bevorzugt 1 : 1,5 bis 1,5 : 1.

Im Folgenden werden die für SLN geeigneten Wirkstoffträger, geeignete Emulgatoren, die vorzugsweise Lamellarstrukturen ausbilden, geeignete pharmazeutische, kosmetische und lebensmitteltechnologische Wirkstoffe und weitere mögliche Inhaltsstoffe der wässrigen Wirkstoffträger-Dispersion näher erläutert.

Als Wirkstoffträgerteilchen werden Teilchen auf Lipidbasis eingesetzt. Hierzu gehören Lipide und lipidähnliche Strukturen. Beispiele geeigneter Lipide sind die Di- und Triglyceride der gesättigten geradkettigen Fettsäuren mit 12 bis 30 Kohlenstoffatomen, wie Laurinsäure, Myristinsäure, Palmitinsäure, Stearinsäure, Arachinsäure, Behensäure, Lignocerinsäure, Cerotinsäure, Melesinsäure, sowie deren Ester mit anderen gesättigten Fettalkoholen mit 4 bis 22, vorzugsweise 12 bis 22 Kohlenstoffatomen wie Laurylalkohol, Myristylalkohol, Cetylalkohol, Stearylalkohol, Arachidylalkohol, Behenylalkohol, gesättigten Wachsalkoholen mit 24 bis 30 Kohlenstoffatomen wie Lignocerylalkohol, Cerylalkohol, Cerotylalkohol, Myrizylalkohol. Bevorzugt sind Di-, Triglyceride, Fettalkohole, deren Ester oder Ether, Wachse, Lipidpeptide oder Mischungen davon. Insbesondere werden synthetische Di- und Triglyceride als Einzelsubstanzen oder in Form einer Mischung, zum Beispiel in Form eines Hartfettes, eingesetzt. Glycerintrifettsäureester sind beispielsweise Glycerintrilaurat, Glycerintrimyristat, Glycerintripalmitat, Glycerintristearat oder Glycerintribehenat. Geeignete Wachse sind beispielsweise Cetylpalmitat und Cera alba (gebleichtes Wachs, DAB 9).

Die Menge der Wirkstoffträgerteilchen, bezogen auf die gesamte wässrige Wirkstoffträger-Dispersion, beträgt vorzugsweise 0,1 bis 30 Gew.-%, besonders bevorzugt 1 bis 10 Gew.-%. Zusätzlich zu den Lipiden können Dispersionsstabilisatoren bzw. Emulgatoren eingesetzt werden. Sie können beispielsweise in Mengen von 0,01 bis 10 Gew.-%, vorzugsweise 0,05 bis 5 Gew.-% eingesetzt werden. Beispiele geeigneter Substanzen sind Tenside, insbesondere Alkyllactylate wie Stearoyllactylat, Isethinonate, Alkylsulfate wie Cetylsulfat, Diamideethersulfate, Alkylpolyglycoside, Phosphorsäureester, Taurate, Sulfosuccinate, Alkylpolyglycoside, Phosphorsäureester, Taurate, Sulfosuccinate, Alkylsarcosinate wie Natriumlaurylsarcosinat und Alkylglutamate wie Natriumlaurylglutamat, ethoxylierte Sorbitanfettsäureester, Blockpolymere und Blockcopolymere (wie zum Beispiel Poloxamere und Poloxamine), Polyglycerinether und -ester, Lecithine verschiedenen Ursprungs (zum Beispiel Ei- oder Sojalecithin), chemisch modifizierte Lecithine (zum Beispiel hydriertes Lecithin) als auch Phospholipide und Sphingolipide, Mischungen von Lecithinen mit Phospholipiden, Sterine (zum Beispiel Cholesterin und Cholesterinderivate sowie Stigmasterin), Ester und Ether von Zuckern oder Zuckeralkoholen mit Fettsäuren oder Fettalkoholen (zum Beispiel Saccharosemonostearat), sterisch stabilisierende Substanzen wie Poloxamere und Poloxamine (Polyoxyethylen-Polyoxypropylen-Blockpolymere), ethoxylierte Sorbitanfettsäureester, ethoxylierte Mono- und Diglyceride, ethoxylierte Lipide und Lipoide, ethoxylierte Fettalkohole oder Fettsäuren und Ladungsstabilisatoren bzw. Ladungsträger wie zum Beispiel Dicetylphosphat, Phosphatidylglycerin sowie gesättigte und ungesättigte Fettsäuren, Natriumcholat, Natriumglykolcholat, Natriumtaurocholat oder deren Mischungen, Aminosäuren oder Peptisatoren wie Natriumcitrat (siehe J. S. Lucks, B. W. Müller, R. H. Müller, Int. J. Pharmaceutics 63, Seiten 183 bis 18 (1990)), viskositätserhöhende Stoffe wie Celluloseether und -ester (zum Beispiel Methylcellulose, Hydroxyethylcellulose, Hydroxypropylcellulose, Natriumcarboxymethylcellulose), Polyvinylderivate wie Polyvinylalkohol, Polyvinylpyrrolidon, Polyvinylacetat, Alginate, Polyacrylate (zum Beispiel Carbopol), Xanthane und Pektine.

Als wässrige Phase A können Wasser, wässrige Lösungen oder Mischungen von Wasser mit wassermischbaren Flüssigkeiten wie Glycerin oder Polyethylenglycol eingesetzt werden. Weitere zusätzliche Komponenten für die wässrige Phase sind beispielsweise Mannose, Glucose, Fructose, Xylose, Trehalose, Mannit, Sorbit, Xylit oder andere Polyole wie Polyethylenglykol sowie Elektrolyte wie Natriumchlorid. Diese zusätzlichen Komponenten können in einer Menge von 1 bis 30 Gew.-%, bezogen auf die wässrige Phase A, eingesetzt werden.

Falls gewünscht, können ferner viskositätserhöhende Stoffe oder Ladungsträger eingesetzt werden, wie Sie in EP-B-0 605 497 beschrieben sind. Als Verdicker können zum Beispiel Polysaccharide, Polyalkylacrylate, Polyalkylcianoacrylate, Polyalkylvinylpyrrolidone, Acrylpolymere, Polymilchsäuren oder Polylactide eingesetzt werden.

Lipide und Emulgatoren werden in einem Gewichtsverhältnis von 50 : 1 bis 2:1, vorzugsweise 15 : 1 bis 30 : 1 eingesetzt. Die Menge an Duftöl bzw. Aroma beträgt vorzugsweise 0,1 bis 30 Gew.-%.
Der mittlere Durchmesser der Wirkstoffteilchen beträgt vorzugsweise 50 bis 1000 nm, besonders bevorzugt 100 bis 500 nm.

In den Zusammensetzungen können die Wasserresistenz und das Spreit- und Haftvermögen durch Silikonzusatz erhöht werden. Geeignete Silikonderivate sind dabei Dimethicon, alkyl- und arylsubstituierte Silikone, aminosubstituierte Silikonöle, Silikon-Copolyole mit Alkylpolyglykosiden, modifizierte Silikonöle usw. Alternativ oder zusätzlich können auch fluorierte Kohlenwasserstoffe eingesetzt werden.

In SLN-Dispersionen ist das Freisetzungsverhalten vom Schmelzpunkt der Lipidteilchen abhängig. Ferner ist das Freisetzungsverhalten von der Zusammensetzung und Menge des eingesetzten Emulgators abhängig.

Gemäß einer Ausführungsform der Erfindung liegen in den SLN-Dispersionen Teilchen auf Lipidbasis mit unterschiedlichen Schmelzpunkten vor. Hierdurch wird eine gezielte Freisetzung, insbesondere kaskadierte Freisetzung der enthaltenen Duftöle und Aromen möglich. Zunächst erweichen die Lipidteilchen mit dem niedrigsten Schmelzpunkt und geben den entsprechenden Wirkstoff frei. Erst zu einem späteren Zeitpunkt erweichen die Lipidteilchen mit höherem Schmelzpunkt. In den Lipidteilchen werden vorzugsweise öllösliche und/oder amphiphile Duftöle oder Aromen gespeichert, während wasserlösliche Duftstoffe in die wässrige Phase eingebracht werden. Beim Einsatz einer derartigen SLN-Dispersion werden zunächst die im Wasser enthaltenen Duftöle und Aromen freigesetzt, während die in den Lipidteilchen vorliegenden Duftöle und Aromastoffe erst nachträglich freigesetzt werden.

Zur weiteren kaskadierten Freisetzung können die SLN-Dispersionen auch mit den nachfolgend beschriebenen Zusammensetzungen, zum Beispiel mit PO- oder POW-Emulsionen kombiniert werden.

Es wurde zudem gefunden, dass Duftstoffe und Aromen in liquidkristalline (flüssigkristalline) Lamellen, die von Tensiden als lyotrope Flüssigkristallphäse gebildet werden, eingebunden werden können und in dieser Form ein Depot darstellen.

Durch die Tenside kann ein unilamellares oder multilamellares System bzw. eine lyotrope flüssigkristalline Mischphase gebildet werden. Diese Zusammensetzungen weisen vorzugsweise mikroskopisch doppelbrechende Grenzflächen auf, die sich von einem Bilayer oder einem Multilayer der Lamellarstrukturen bzw. LC-Phasentensid ableiten.

Es werden dabei solche Tenside eingesetzt, die hydrophile und hydrophobe Reste aufweisen und zur Ausbildung von lyotropen lamellaren liquidkristallinen Phasen fähig sind. Die Bildung von liquidkristallinen Strukturen hängt im Wesentlichen von der Geometrie der Tenside ab. Dabei spielt das Verhältnis von hydrophilem zu hydrophobem Rest eine wichtige Rolle. Tenside mit einer raumerfüllenden hydrophilen Gruppe und kleinem hydrophoben Rest bilden häufig Mizellen aus. Mizellen liegen jedoch in einem dynamischen Gleichgewicht vor und bauen sich ständig ab und wieder auf. Daher sind Mizellen nicht als Speicher für andere Inhaltsstoffe geeignet. Mit kleiner werdendem hydrophilen Rest bilden die Tenside stäbchenförmige Mizellen, Vesikel-Doppelschichten und Sandwich-Doppelschichten aus. Es werden Tenside eingesetzt, die in einer lyotropen lamellaren liquidkristallinen Phase vorliegen können. Im lyotropen Zustand werden zwischen den hydrophoben Resten bzw. Köpfen der Tenside Duftstoffe gespeichert Der hydrophile Teil des Tensids kann je nach gewünschter Haftung zu einem späteren Substrat variiert werden. Beispielsweise kann der hydrophobe Teil variiert werden zur Haftung an der menschlichen Haut oder an Textilfasern.

Geeignete Tenside, die lyotrope lamellare liquidkristalline Phasen ausbilden, sind dem Fachmann bekannt. Es können natürliche oder synthetische Produkte eingesetzt werden. Auch der Einsatz von Tensidgemischen ist möglich. Beispiele geeigneter Tenside sind die physiologischen Gallensalze wie Natriumcholat, Natriumdehydrocholat, Natriumdeoxucholat, Natriumglykocholat, Natriumtaurocholat. Tierische und pflanzliche Phospholipide wie Lecithine mit ihren hydrierten Formen sowie Polypeptide wie Gelatine mit ihren modifizierten Formen können ebenso verwendet werden.

Als synthetische grenzflächenaktive Substanzen eignen sich die Salze der Sulfobernsteinsäureester, Polyoxyethylensäurebetanester, Säurebetanester und Sorbitanether, Polyoxiethylenfettalkoholether, Polyoxiethylenstearinsäureester sowie entsprechende Mischkondensate von Polyoxiethylen- mit Polyoxipropylenethem, ethoxylierte gesättigte Glyceride, partielle Fettsäure-Glyceride und Polyglycide. Beispiele geeigneter Tenside sind Biobase^{®} EP und Ceralution^{®} H.

Beispiele geeigneter Tenside sind ferner Glycerinester, Polyglycerinester, Sorbitanester, Sorbitolester, Fettalkohole, Propylenglykolester, Alkylglucosidester, Zuckerester, Lecithin, Silikoncopolymere, Wollwachs und deren Mischungen oder Derivate davon. Glycerinester, Polyglycerinester, Alkoxylate und Fettalkohole sowie Isoalkohole können sich beispielsweise ableiten von Rhizinusfettsäure, 12-Hydroxystearinsäure, Isostearinsäure, Ölsäure, Linolsäure, Linolensäure, Stearinsäure, Myristinsäure, Laurensäure und Caprinsäure. Neben den genannten Estern können auch Succinate, Amide oder Ethanolamide der Fettsäuren vorliegen. Als Fettsäurealkoxylate kommen insbesondere die Ethoxylate, Propoxylate oder gemischten Ethoxylate/Propoxylate in Betracht.

Die Auswahl der Tenside kann je nach gewünschtem Einsatzgebiet erfolgen. Beim Einsatz im Waschmittelbereich werden beispielsweise vorzugsweise kationische Tenside eingesetzt. Für auf die menschliche Haut aufzubringende Zusammensetzungen kommen insbesondere Lactylate, Glutamate, Diamid-Ethersulfate, Ethoxylate von Alkoholen oder Glykolen, Betaine, amphiphile Coemulgatoren wie Sorbitanmonostearat und Fettalkohole, Fettsäurekondensate, Sarkosinate, Proteinfettsäurekondensate, Sulfosuccinate und Ethercarboxylate zum Einsatz.

Je nach Form und Größe der ausgebildeten Lamellen können die Zusammensetzungen klar erscheinen oder einen Perleffekt zeigen. Teilchen mit einer mittleren Teilchengröße im Bereich von 100 nm erscheinen klar, während lange Lamellen in der Regel einen Perleffekt zeigen.

Die lyotrope lamellare liquidkristalline Phase wird dabei vorzugsweise unter Einsatz von Wasser, Alkoholen, Polyolen oder Gemischen davon ausgebildet. Hierbei orientieren sich die hydrophilen Anteile der Tenside zur Wasser-, Alkohol-, Polyol-Phase oder Mischphase davon aus, während die hydrophoben Anteile in der lamellaren Struktur zueinander weisen. In dieser Anordnung werden nun die Duftstoffe bzw. Aromen in die Räume zwischen den hydrophoben Resten und/oder der Tenside eingebracht und gespeichert. Die Menge des speicherbaren Duftstoffs hängt dabei von der Art des eingesetzten Tensids und dem Aufbau der liquidkristallinen Phase ab. In der Regel liegen Tensid und Duftstoff bzw. Aroma in einem Gewichtsverhältnis von 1 : 100 bis 100 : 1 vorzugsweise 1 : 20 bis 20 : 1, besonders bevorzugt 1 : 5 bis 5 : 1 vor.

Das Freisetzungsverhalten der Duftstoffe/Aromen aus den liquidkristallinen Phasen hängt dabei vom Schmelzpunkt der lamellaren Struktur und der Art des hydrophoben Restes im Tensid ab. Bei Verwendung von Polyolen anstelle von Wasser zur Ausbildung der liquidkristallinen Phase kann die Flüchtigkeit und Verfügbarkeit vermindert werden, so dass sich ein länger anhaltender Speichereffekt ergibt. Bei Rinse-off-Produkten wie Waschmitteln, Shampoos, Duschgelen oder Seifen, wird der Hauptteil der Zusammensetzung abgespült und vom Substrat abgetragen. Die erfindungsgemäßen Zusammensetzungen erlauben das Aufziehen der Duftstoffe und Aromen auf das Substrat, beispielsweise die Haut oder Textilien, so dass eine größere Menge an Duftstoff auf dem Substrat verbleibt.

Eine kaskadierte Freisetzung kann insbesondere auch bei Leave-on-Produkten wichtig sein, sofern unterschiedliche Duftöle oder Aromastoffe zu unterschiedlichen Zeitpunkten freigesetzt werden. Es wird damit auf die Sinnesorgane jeweils ein neuer Reiz ausgeübt, so dass die Wahrnehmung verstärkt wird. Bei Vorliegen nur eines Reizes tritt üblicherweise eine Sättigung ein, die die Wahrnehmung des Duftöls oder Aromas beeinträchtigt. Mit Hilfe der erfindungsgemäßen Zusammensetzungen dauert das Geruchs- bzw. Geschmacksempfinden an.

Die unterschiedlichen Zusammensetzungen wie Emulsionen, Dispersionen oder SLN-Dispersionen können auch gemischt bzw. kombiniert werden, um aus der Kombination neue Eigenschaftsprofile zu erhalten.

Insbesondere SLN-Dispersionen oder Lipidteilchen führen zu einem optimalen Oxidationsschutz für die Duftstoffe und Aromen, da die Duftstoffe und Aromen im Festkörper eingebettet vorliegen und so gegenüber dem Luftzutritt abgeschlossen sind. Gegebenenfalls können in den Zusammensetzungen Amphiphile und Antioxidantien mit verwendet werden, um die Oxidationsstabilität weiter zu erhöhen.

Unter Duftstoffen und Aromen werden erfindungsgemäß sowohl Duftöle (Flagrance) wie auch Aromastoffe (Flavour) verstanden. Es handelt sich hierbei um Riechstoffe, speziell Duftstoffe. Grundstoffe der Duftstoffe sind in der Regel etherische Öle, Blütenöle, Extrakte aus pflanzlichen und tierischen Drogen, aus Naturprodukten isolierte, chemisch veränderte (halbsynthetische) sowie rein synthetisch gewonnene Riechstoffe. Zu den Duftstoffen werden erfindungsgemäß auch Geschmacksstoffe gezählt.

Die Duftstoffe und Aromen können dabei aus einer Vielzahl pflanzlicher Ausgangsmaterialien stammen. Beispielsweise können genannt werden: Blüten, beispielsweise von Lavendel, Rosen, Jasmin, Neroli; Stängel und Blätter, beispielsweise von Geranium, Patchouli, Petitgrain, Früchte wie Anis, Koriander, Kümmel, Wacholder; Fruchtschalen, beispielsweise von Agrumen wie Bergamotte, Zitronen, Orangen; Samen wie Macis, Angelika, Sellerie, Kardamom; Wurzeln wie Angelika, Costus, Iris, Calmus; Holz wie Sandel-, Guajak-; Zedern-, Rosenholz; Kräuter und Gräser wie Estragon, Limonengras, Salbei, Thymian; Nadeln und Zweige, beispielsweise von Fichten, Tannen, Kiefern, Latschen; Harze und Balsame, beispielsweise aus Galvanum, Elemi, Benzoe, Myrrhe, Olibanum, Opoponax.

Tierische Rohstoffe sind beispielsweise Ambra, Moschus, Zibet, Castoreum.

Beispiele halb synthetischer Riechstoffe sind Isoeugenol, Vanillin, Hydroxycitronellal, Citronellol, Geranylacetat, Jonone und Methyljonone. Die vollsynthetischen Riechstoffe oder Duftstoffe sind sehr vielfältig und orientieren sich häufig an natürlichen Stoffen. Für eine Beschreibung der Duftstoffe kann beispielsweise auf Römpp, Chemielexikon, 9. Auflage, Stichworte "Parfums", "Riechstoffe", "Duftstoffe", verwiesen werden. Weitere geeignete Duftstoffe und Aromen sind dem Fachmann bekannt.

Neben Duftstoffen und Aromen können auch weitere Wirkstoffe in die liquidkristallinen Phasen eingebracht werden. Bei Kosmetika handelt es sich insbesondere um kosmetische oder pharmazeutische Wirkstoffe, zum Beispiel Antioxidantien, bei waschmitteltechnologischen Zusammensetzungen beispielsweise um Additive wie Weichspülaktivkomponenten.

Die liquidkristalline Phase wird zum Beispiel unter Einsatz von Wasser, Etheralkoholen, Etherpolyolen, Esterpolyolen, aminofunktionellen Polyolen oder Gemischen davon ausgebildet.

Der Etheralkohol weist vorzugsweise die nachstehende allgemeine Formel (I) auf

R¹-O-[EO-]ₙ[PO-]ₘR² (I)

mit der Bedeutung
- R¹: C₁₋₄-Alkyl,
- R²: Wasserstoff oder C₁₋₄-Alkyl,
- n: im Mittel 1 bis 100,
- m: im Mittel 0 bis n/2
- EO, PO: von Ethylenoxid und Propylenoxid abgeleitete Grundbausteine, die beim Vorliegen beider Grundbausteine in beliebiger Reihenfolge vorliegen können.

In den Etheralkoholen der allgemeinen Formel (I) können von Ethylenoxid und gegebenenfalls zusätzlich von Propylenoxid abgeleitete Grundbausteine vorliegen. Diese Grundbausteine haben die Strukturen -CH₂-CH₂-O-, -CH₂-CH(CH₃)-O- und -CH(CH₃)-CH₂-O-. Wenn die beiden von Ethylenoxid und Propylenoxid abgeleiteten Grundbausteine vorliegen, können sie in beliebiger Reihenfolge vorliegen. Dies bedeutet, dass jeweils ein oder mehrere von Ethylenoxid und Propylenoxid abgeleitete Blöcke miteinander verbunden sein können. Ferner können die von Ethylenoxid und Propylenoxid abgeleiteten Einheiten auch alternierend oder statistisch vorliegen. Die zwischen diesen Formen möglichen kontinuierlichen Übergänge sind ebenfalls erfindungsgemäß möglich.

In der allgemeinen Formel (I) ist der Anteil der von Propylenoxid abgeleiteten Grundbausteinen maximal ein Bruchteil der Menge von Ethylenoxid abgeleiteten Grundbausteine. Während im Mittel 1 bis 100, vorzugweise 2 bis 70, besonders bevorzugt 3 bis 50, insbesondere 5 bis 15 von Ethylenoxid abgeleitete Grundbausteine vorliegen, liegen 0 bis n/2, vorzugsweise 0 bis n/4, besonders bevorzugt 0 bis n/8 von Propylenoxid abgeleitete Grundbausteine im Mittel vor. Sofern von Propylenoxid abgeleitete Grundbausteine vorliegen, beträgt ihre Menge vorzugsweise n/10 bis n/4, besonders bevorzugt n/8 bis n/5. Bei den Zahlen n und m handelt es sich um Mittelwerte, da sich bei der Alkoxylierung in der Regel eine Verteilung des Alkoxylierungsgrades einstellt. Deshalb sind auch ungradzahlige Werte für n und m möglich. Die Breite der Verteilung des Alkoxylierungsgrades hängt unter anderem auch vom verwendeten Alkoxylierungskatalysator ab. Es ist auch möglich, diskrete Alkoxylierungsgrade oder sehr eng verteilte Verteilungen des Alkoxylierungsgrades einzustellen.

R¹ ist ein C₁₋₄-Alkylrest, vorzugsweise C₁₋₃-Alkylrest, besonders bevorzugt C₁₋₂-Akylrest, insbesondere ein Methylrest Propylreste umfassen n-Propyl und iso-Propyl, während Butylreste n-Butyl, iso-Bütyl, tert.Butyl umfassen.

R² bedeutet Wasserstoff oder einen Rest, wie er vorstehend für R¹ definiert ist. Dabei ist die Bedeutung von R² unabhängig von der Bedeutung des Restes R¹. Besonders bevorzugt ist R² Wasserstoff. Der in der Beschreibung verwendete Ausdruck "Etheralkohol" umfasst sämtliche Verbindungen der allgemeinen Formel (I), d.h. auch die Fälle, in denen R² kein Wasserstoffatom ist und somit keine freien Hydroxylgruppen im Molekül vorliegen.

Bevorzugt handelt es sich beim Etheralkohol um ein Methanolethoxylat mit 5 bis 15 Ethylenoxid-Einheiten.
Speziell bevorzugt werden . Polyethylenglykolmonomethylether (12EO) und Polyethylenglykolmonomethylether (7EO) eingesetzt. Hierbei handelt es sich um reine Methylalkoholethoxylate. Derartige Verbindungen sind an sich bekannt und wurden bisher für die Herstellung von methylendgruppenverschlossenen Fettsäurepolyethylenglykolestern eingesetzt. Die Verbindungen sind handelsüblich.

Als Polyole können die üblichen bekannten Polyole wie Propylenglykol, Butylenglykol, Ethylenglykol, Polyalkylenglykol, Glycerin, Polyglycerin, Glycoside, Sorbit, Mannit, Pentaerithrit, Trimethylopropan oder Mischungen davon eingesetzt werden. Als Polyalkylenglykole kommen insbesondere Polyethylenglykol und Polypropylenglykol in Frage. Weitere geeignete Polyole sind dem Fachmann bekannt, beispielsweise aromatische Polyole wie Emodin/Aloe Vera.

Geeignete Esterpolyole leiten sich ab von
Glycerin, C₂₋₂₀-Alkylenglycol, vorzugsweise C₂₋₁₀-Akylenglykol, insbesondere C₂₋₈-Alkylenglykol, zum Beispiel Propylenglykol, Butylenglykol und speziell Ethylenglykol, Poly(C₂₋₂₀-alkylen)glykol, vorzugsweise Poly(C₂₋₁₀-alkylen)glykol, insbesondere Poly(C₂₋₈-alkylen)glykol, mit einem oder mehreren unterschiedlichen Alkylenresten, zum Beispiel Polyethylenglykol, in dem bis zu 30% der Ethylenoxyeinheiten durch Propylenoxyeinheiten ersetzt sein können, Polyglycerinen, Sorbitol, Pentaerythritol als Polyolkomponente und
aliphatischen C₃₋₆-Carbonsäuren, die neben einer Carboxylgruppe mindestens eine weitere funktionelle Gruppe, ausgewählt aus Hydroxyl- und Carboxylgruppen, aufweisen, als Säurekomponente.

Die Esterpolyole, die erfindungsgemäß eingesetzt werden können, leiten sich von den Polyolkomponenten und Säurekomponenten ab, die miteinander verestert sind. Im Polyethylenglykol liegen vorzugsweise 1 bis 600, besonders bevorzugt 1 bis 100 Ethylenoxyeinheiten vor.

Vorzugsweise enthalten die Carbonsäuren nur Kohlenstoff, Wasserstoff und Sauerstoff als Atome. Die Kohlenstoffkette kann dabei frei von Doppelbindungen sein oder eine Doppelbindung enthalten. Beispiele geeigneter Säurekomponenten sind Milchsäure, Maleinsäure, Weinsäure und Citronensäure.
In den Esterpolyolen können eine, zwei, drei oder mehr, beispielsweise alle Hydroxylgruppen der Polyolkomponente verestert vorliegen. Ebenso können eine, zwei, mehrere oder alle Carboxylgruppen der Säurekomponente in Esterform vorliegen.

Die erfindungsgemäß eingesetzten Esterpolyole können bei Raumtemperatur (25°C) in fester, glasähnlicher, zähflüssiger oder flüssiger Form vorliegen. Durch Mischung mehrerer Esterpolyole können die Eigenschaften gezielt eingestellt werden. Beispielsweise liegen Lactate in der Regel flüssig vor, während Citrate in einem glasähnlichen Zustand vorliegen und erst bei einer Temperatur von etwa 50°C zähflüssig werden. Ein Gemisch aus Citrat und Lactat im Verhältnis 1:1 ist glasähnlich. Ein Gemisch von Citrat und Lactat im Gewichtsverhältnis 1:2 ist demgegenüber zähflüssig. Häufig sind die Esterpolyole bei Raumtemperatur glasklar und fest.

Die Esterpolyole können in Kombination mit aminofunktionellen Polyolen, Etheralkoholen oder beiden Verbindungen eingesetzt werden. Der Anteil der Esterpolyole an derartigen Gemischen beträgt vorzugsweise mindestens 10 Gew.-%, besonders bevorzugt mindestens 15 Gew.-%, insbesondere mindestens 20 Gew.-%.

Als aminofunktionelles Polyol kann jedes geeignete aminofunktionelle Polyol eingesetzt werden, das einen Schmelzpunkt von weniger als 100 °C, vorzugsweise von weniger als 50 °C aufweist. Der Ausdruck "aminofunktionelle" Polyole bedeutet, dass im Polyol mindestens eine Amingruppe oder Amidgruppe vorliegt. Das Polyol weist zudem mindestens zwei Hydroxylgruppen auf. Es kann sich dabei um ein aliphatisches, aromatisches oder aromatisch/aliphatisches Polyol handeln. Vorzugsweise handelt es sich um ein aliphatisches Polyol. Besonders bevorzugt liegen im Polyol 2 bis 10, besonders bevorzugt 2 bis 5, insbesondere 2 bis 3 Hydroxylgruppen vor. Die aminofunktionellen Polyole können über die genannten funktionellen Gruppen hinaus Carbonylgruppen, Carboxylgruppen, Thiolgruppen und Kohlenstoff-Kohlenstoff-Doppelbindungen oder - Dreifachbindungen aufweisen. Sie können zudem Ethergruppen aufweisen. Ein Teil der im Molekül vorliegenden Hydroxylgruppen kann derart verethert sein, unter der Voraussetzung, dass mindestens zwei freie Hydroxylgruppen im Molekül vorliegen. Vorzugsweise enthalten die erfindungsgemäß eingesetzten aminofunktionellen Polyole eine oder zwei Amidgruppen.

Bevorzugt werden erfindungsgemäß aminofunktionelle Polyole eingesetzt, die zwei oder drei Hydroxylgruppen und eine oder zwei Amidgruppen aufweisen. Sie können ferner eine zusätzliche Thiolgruppe oder Carboxylgruppe aufweisen. Vorzugweise enthalten die Verbindungen 5 bis 15 Kohlenstoffatome, besonders bevorzugt 7 bis 12 Kohlenstoffatome.

Vorzugsweise ist das aminofunktionelle Polyol ein Pantothenderivat der allgemeinen Formel (II)

HO-CH₂-C(CH₃)₂-CH(OH)-CO-NH-CH₂-CH₂-R (II)

- mit R: COOR¹, CH₂-OR¹, CO-NH-CH₂-CH₂-SR¹
- R¹: H, C₁₋₁₂-Alkyl, Phenyl.

Vorzugsweise ist R ein Rest CH₂-OR¹. Vorzugsweise ist R¹ Wasserstoff oder C₁₋₆-Alkyl, insbesondere Wasserstoff oder C₁₋₃-Alkyl.

Besonders bevorzugt werden Pantothensäure, Panthenol oder Panthetein eingesetzt. Die Pantothenderivate liegen vorzugsweise in der (R)-Form vor. Pantothensäure kann auch in Form der Salze eingesetzt werden, speziell bevorzugt ist das Pantothenderivat (R)-Panthenol.

Die erfindungsgemäß bevorzugt eingesetzten Verbindungen weisen einen Molekülabschnitt auf, der sich von Pantoinsäure ableitet, sowie einen Molekülabschnitt, der sich von β-Alanin ableitet, sofern es sich um eine Pantothensäure-vergleichbare Verbindung handelt.

Panthenol und Pantothensäure sind aus dem medizinischen Bereich zur Wundheilung sowie aus Haarbehandlungsmitteln und Futterzusätzen bekannt. Die Herstellung kann über eine Biosynthese aus 2-Oxo-3-methylbutansäure oder aus Pantolacton erfolgen. Weitere Herstellungsverfahren sind allgemein bekannt. Es kann ferner auf Beilstein EIV 4, 2571, Beilstein EV 18/1, 22 und Beilstein E IV 4, 2569f sowie Isler et al., Vitamine II, Seiten 309 bis 339, Thieme-Verlag, Stuttgart, 1988 verwiesen werden. Es gibt zudem eine umfängliche patentliteratur, die sich mit dem Einsatz von Pantothenderivaten, insbesondere von Panthenol in kosmetischen und pharmazeutischen Zusammensetzungen befasst.

Vorzugsweise liegen in den erfindungsgemäßen Duftstoff Depots 1 bis 95 Gew.-%, besonders bevorzugt 10 bis 50 Gew.-% Wasser, Alkohole, Polyole oder Gemische davon, 0,5 bis 30 Gew.-%, besonders bevorzugt 2 bis 20 Gew.-% Tenside und 0,1 bis 60 Gew.-%, besonders bevorzugt 2 bis 30 Gew.-% Duftstoffe und/oder Aromen vor, bezogen auf das Gesamtgewicht der Zusammensetzung, das 100 Gew.-% ergibt. Das Duftstoff-Depot bzw. die Zusammensetzung kann dabei neben den Duftstoffen und Aromen, Tensiden und der Wasser-, Alkohol- oder Polyolphase noch weitere übliche Inhaltstoffe enthalten. Es kann jedoch auch aus den drei genannten Komponenten in den angegebenen Mengen bestehen.

Beschrieben werden auch Zusammensetzungen, die hergestellt werden, indem man das Tensid mit dem in flüssiger Form vorliegenden Wasser, Alkohol, Polyol oder Gemisch davon unter Ausbildung einer lyotropen lamellaren liquidkristallinen Phase vermischt und sodann das Duftstoff und/oder Aroma einmischt. Dabei werden mindestens 80 %, vorzugsweise mindestens 90 %, insbesondere mindestens 95 %, vorzugsweise die Gesamtmenge des Duftstoffs bzw. Aromas in die liquidkristalline Struktur eingebaut, so dass die Duftstoffe bzw. Aromen in den hydrophoben Anteilen der Tenside gespeichert vorliegen. Wie beschrieben kann das Erscheinungsbild des Duftstoff/Aroma-Depots gesteuert werden, so dass das Depot klar oder mit einem Effekt wie ein Perleffekt erscheinen kann.

Es liegen erfindungsgemäß keine Duftstoff/Aroma-Tröpfchen in den Zusammensetzungen vor, und es liegt auch keine Duftstoff/Aroma-Emulsion vor. Das Duftstoff/Aroma wird an einer Grenzfläche in den LC-Strukturen mit höchstem Absorptionsanteil deponiert.

Die erfindungsgemäße Zusammensetzung kann zur retardierten Freisetzung des Duftstoffs/Aromas in kosmetischen, pharmazeutischen, lebensmitteltechnologischen oder waschmittel-technologischen Zusammensetzungen verwendet werden. Bei den kosmetischen und pharmazeutischen Zusammensetzungen kann es sich beispielsweise um Rinse Off-Produkte oder Leave On-Produkte handeln. Bei den Rinse Off-Produkten handelt es sich insbesondere um Produkte zur Haar- und Hautreinigung wie Seifen, Shampoos, Duschöle, Duschbäder oder Badezusätze. Bei den Leave On-Produkten handelt es sich um Produkte, die auf die Haut aufgetragen werden und auf der Haut verbleiben sollen. Es handelt sich dabei insbesondere um Gele, Cremes, Lotionen wie Lichtschutzmittel, insbesondere Sonnencremes, Feuchtigkeitscremes und andere derartige Produkte.

Beim Auftrag der Zusammensetzung auf die Haut verdunstet das Wasser oder Polyol, und die LC-Strukturen werden auf der Haut gespreitet. Das Freisetzungsverhalten ist dabei abhängig vom Schmelzpunkt der Lamellen und der Art des hydrophoben Restes der Tenside. Das Freisetzungsverhalten kann damit in weiten Grenzen gesteuert werden. Es kommt dabei zu einer größeren Verfügbarkeit des Duftstoffs bzw. Aromas auf der Haut im Vergleich zu bislang verwendeten Zusammensetzungen und Aufbringungsmechanismen für Duftstoffe/Aromen. Aufgrund des großen Anteils des aufziehenden Duftstoffs/Aromas sind nur geringe Einsatzmengen des Duftstoffs nötig. Eine gezielte Freisetzung auf der Haut ist durch Kombination von Wasser mit Polyolen möglich. Die Spreitung und Fixierung kann dabei in geeigneter Weise den Erfordernissen angepasst werden.

Über die Speicherwirkung hinaus erlaubt die erfindungsgemäße Vorgehensweise einen weitgehenden Schutz der Duftstoffe vor dem oxidativen Zerfall. Gegebenenfalls können noch weitere Antioxidantien zugesetzt werden. Auch ohne den Zusatz von Antioxidantien ist das DuftstofF/Aroma in den erfindungsgemäßen Depots wesentlich besser gegenüber der Oxidation geschützt als in herkömmlichen Darreichungsformen.

Die Erfindung begriff auch kosmetische, pharmazeutische, lebensmitteltechnologische oder waschmitteltechnologische Zusammensetzungen, die eine wie beschriebene und beanspruchte Zusammensetzung enthalten. Die Zusammensetzungen können beispielsweise in Form einer Emulsion oder Dispersion vorliegen. Dabei können sie in Form beliebiger Emulsionen, multipler Emulsionen, Vesikeln oder Dispersionen vorliegen. Vorzugsweise liegen sie in Form einer PO-Emulsion oder POW-Emulsion vor. Auch Gemische der Emulsionen bzw. Dispersionen sind möglich.

In Polyol-in-Öl-Emulsionen (PO-Emulsionen) können eine oder mehrere Polyolphasen vorliegen. Die Polyolphase ist dabei vorzugsweise nicht mit Öl mischbar. Als Öl können in der Ölphase alle bekannten geeigneten Öle und deren Gemische eingesetzt werden. Beispiele geeigneter Öle sind Silikonöle und Derivate davon, die linear oder zyklisch sein können, natürliche Esteröle wie Traubenkernöl, Olivenöl oder Sonnenblumenöl, synthetische Esteröle wie Neutralöle, die linear oder verzweigt sein können, Paraffinöl und Isoparaffinöle, Esteröle, beispielsweise der Citrate, Lactate, Aleate, Salicylate, Cinnamate oder von Kampherderivaten, Triglyceride, Fettalkohole oder Mischungen davon.

In den PO-Emulsionen beträgt das Gewichtsverhältnis von Polyolphase zu Ölphase vorzugsweise 10 : 90 bis 90 : 10, besonders bevorzugt 25 : 75 bis 75 : 25 und insbesondere 40 : 60 bis 60 : 40.

Zur Ausbildung der PO-Emulsionen können die vorstehend genannten Emulgatoren eingesetzt werden.
Die Menge des Emulgators kann den praktischen Erfordernissen angepasst werden. Vorzugsweise wird der Emulgator in einer Menge von 0,1 bis 20 Gew.-%, besonders bevorzugt 0,5 bis 15 Gew.-%, insbesondere 1 bis 8 Gew.-%, bezogen auf die gesamte P/O-Emulsion, eingesetzt. Fallweise können hiervon abweichende Dosierungen erforderlich sein.

Die Polyolphase kann einen in der Phase gelösten kosmetischen und/oder pharmazeutischen Wirkstoff enthalten. Es kann sich auch um einen waschmitteltechnologischen, lebensmitteltechnologischen oder agrartechnologischen Wirkstoff handeln.

Bei den Wirkstoffen handelt es sich vorzugsweise um in lipophilen und hydrophilen Medien unzureichend lösliche oder unlösliche organische Verbindungen. Die Verbindungen sind dabei insbesondere in Wasser und Öl unzureichend löslich oder unlöslich. Es können beliebige geeignete Wirkstoffe eingesetzt werden, sofern sie sich in dem aminofunktionellen Polyol bzw. der das aminofunktionelle Polyol enthaltenden Polyolphase lösen. Geeignete Wirkstoffe sind beispielsweise Dichlorphenac, Ibuprofen, Acetylsalicylsäure, Erythromycin, Ketoprofen, Cortison, Glucocorticoide.

Weiterhin geeignet sind kosmetische Wirkstoffe, die insbesondere oxidations- oder hydrolyseempfindlich sind wie beispielsweise Polyphenole. Hier seien genannt Catechine (wie Epicatechin, Epicatechin-3-gallat, Epigallocatechin, Epigallocatechin-3-gallat), Flavonoide (wie Luteolin, Apigenin, Rutin, Quercitin, Fisetin, Kaempherol, Rhametin), Isoflavone (wie Genistein, Daidzein, Glycitein, Prunetin), Cumarine (wie Daphnetin, Umbelliferon), Emodin, Resveratrol, Oregonin.

Geeignet sind Vitamine wie Retinol, Tocopherol, Ascorbinsäure, Riboflavin, Pyridoxin.

Geeignet sind ferner Gesamtextrakte aus Pflanzen, die unter anderem obige Moleküle oder Molekülklassen enthalten.

Die vorstehend beschriebene P/O-Emulsion kann auch in Wasser oder eine Wasser-in-Öl-Emulsion emulgiert werden. Dabei resultiert eine Polyol-in-Öl-in-Wasser-Emulsion (P/O/W-Emulsion), die mindestens eine wie vorstehend beschriebene Emulsion und zusätzlich mindestens eine wässrige Phase enthält. Derartige multiple Emulsionen können im Aufbau den in DE-A-43 41 113 beschriebenen Emulsionen entsprechen, wobei die Polyolkomponente variiert ist. Der Aufbau der Polyol-in-Öl-Emulsion kann dem Aufbau der in DE-A-43 41 114 beschriebenen Emulsionen entsprechen, wobei die beschriebene Polyolphase als Polyolphase eingesetzt wird.

Beim Einbringen der P/O-Emulsion in Wasser oder wässrige Systeme kann das Gewichtsverhältnis der einzelnen Phasen in weiten Bereichen variiert werden. Vorzugsweise beträgt in der letztendlich erhaltenen P/O/W-Emulsion der Gewichtsanteil der P/O-Emulsion 0,01 bis 80 Gew.-%, besonders bevorzugt 0,1 bis 70 Gew.-%, insbesondere 1 bis 30 Gew.-%, bezogen auf die gesamte P/O/W-Emulsion.

Beim Einbringen der P/O-Emulsion in eine O/W-Emulsion beträgt der Anteil der P/O-Emulsion vorzugsweise 0,01 bis 60 Gew.-%, besonders bevorzugt 0,1 bis 40 Gew.-%, insbesondere 1 bis 30 Gew.-%, bezogen auf die letztendlich erhaltene P/O/W-Emulsion. In der O/W-Emulsion, die hierzu verwendet wird, beträgt der Ölanteil vorzugsweise 1 bis 80 Gew.-%, besonders bevorzugt 1 bis 30 Gew.-%, bezogen auf die eingesetzte O/W-Emulsion.

Die einzelnen Phasen der Emulsionen können noch übliche für die einzelnen Phasen bekannte Inhaltsstoffe aufweisen. Beispielsweise können die einzelnen Phasen weitere in diesen Phasen lösliche pharmazeutische oder kosmetische, waschmitteltechnologische, lebensmitteltechnologische oder agrartechnologische Wirkstoffe enthalten. Die wässrige Phase kann beispielsweise organische lösliche Lichtschutzfilter, hydrophil gecoatetes Mikropigment, Elektrolyte, Alkohole usw. enthalten. Einzelne oder alle der Phasen können zudem Feststoffe enthalten, die vorzugsweise ausgewählt sind aus Pigmenten, Mikrosphären, Silikagel und ähnlichen Stoffen. Die Ölphase kann beispielsweise organisch modifizierte Tonmineralien, hydrophob gecoatete Pigmente, organische öllösliche Lichtschutzfilter, öllösliche kosmetische Wirkstoffe, Wachse, Metallseifen wie Magnesiumstearat, Vaseline oder Gemische davon enthalten. Als Pigmente können Titandioxid, Zinkoxid und Bariumsulfat genannt werden. Insbesondere Titandioxid oder Zinkoxid sind in der Kosmetik als Lichtschutzfilter gebräuchlich und lassen sich mittels der Emulsionen besonders glatt und gleichmaßig auf die Haut auftragen. Mikrosphären oder Silikagel können als Träger für Wirkstoffe eingesetzt werden, und Wachse können beispielsweise als Grundlage für Polituren verwendet werden.

Die Wasserphase kann darüber hinaus Glycerin, Polyethylenglykol, Propylenglykol, Ethylenglykol und ähnliche Verbindungen sowie Derivate davon enthalten.
Die Verwendung von üblichen Hilfs- und Zusatzstoffen in den Emulsionen ist dem Fachmann bekannt.

Die Herstellung der P/O-Emulsionen kann nach bekannten Verfahren erfolgen, wie sie beispielsweise in DE-A-43 41 114 und DE-A-43 41 113 beschrieben sind. Zur Herstellung werden üblicherweise die Polyolphase und die Ölphase, die jeweils Emulgator enthalten können, getrennt auf eine Temperatur im Bereich von 20 bis 90 °C erwärmt und anschließend unter Rühren zusammengegeben.

Die Emulsionen können in Abhängigkeit von der Zusammensetzung, vom Phasenvolumenverhältnis und dem gegebenenfalls vorhandenen Feststoffanteil als feste oder fließfähige Emulsionen hergestellt werden und vorliegen. Es handelt sich dabei um sehr stabile Emulsionen, die unter normalen Handhabungsbedingungen eine hohe Langzeitstabilität aufweisen. Sie erfüllen insbesondere die üblichen Stabilitätsanforderungen im Temperaturbereich von -5 °C bis + 45 °C. Die in der Emulsion vorliegenden Tröpfchen sind dabei sehr beständig, weshalb die Emulsionen insbesondere als Träger für viele Arten von Wirkstoffen geeignet sind.

Die mit Hilfe der genannten Emulgatoren hergestellten Emulsionen können durch einen einfachen Mischvorgang unter Rühren erhalten werden, wobei die Stabilität der Emulsionen durch die eingetragene Rührwerksenergie und die Art des Rührwerkzeugs in der Regel kaum oder nicht beeinflusst wird. Zur Herstellung der Emulsion kann jedes geeignete handelsübliche Rührwerk eingesetzt werden.

Die Emulsionen werden bevorzugt in kosmetischen und/oder pharmazeutischen Zusammensetzungen oder auch in waschmitteltechnologischen, lebensmitteltechnologischen oder agrartechnologischen verwendet. Damit werden auch derartige kosmetische und/oder pharmazeutische Zusammensetzungen beschrieben, die mindestens eine der genannten Emulsionen enthalten. Bei den kosmetischen und/oder pharmazeutischen Zusammensetzungen kann es sich um Hand- oder Körperlotionen, Öle, Salben, Pasten, Gele, Lippenpflegeprodukte, Gesichtspflegeprodukte und ähnliche Zusammensetzungen handeln. Die Zusammensetzungen können dabei in fester, flüssiger oder Aerosolform zum Einsatz kommen.

In Kombination mit Wasser oder Polyolen können Rinse Off- und Leave On-Produkte erhalten werden. Die Duftstoffe sind häufig polyollöslich. In einer POW-Emulsion können hautaffine Polyole mit dem beschriebenen Duftstoff/Aroma-Depot und gegebenenfalls Antioxidantien eine retardierte Freisetzung des Parfums/Aromas auf der Haut bewirken. Durch die retardierte Freisetzung ist ein längeres Geruchserlebnis der Zusammensetzungen möglich. Wird im Vergleich dazu ein Duftöl als Emulsion direkt auf die Haut aufgebracht, kommt es zu einer Filmbildung und damit zu einer sehr schnellen Freisetzung, so dass nur ein kurzes Geruchserlebnis erreicht wird.

Beschrieben wird auch ein Verfahren zur Herstellung einer multiplen Dispersion durch Vermischen einer Dispersion, die wie vorstehend beschrieben hergestellt wurde, mit einer weiteren Polyol- oder Ölphase, sowie eine entsprechend hergestellte multiple Dispersion. Multiple Emulsionen sind beispielsweise in DE-A-43 41 113 beschrieben.

Weitere Inhaltsstoffe der erfindungsgemäß hergestellten wässrigen wirkstoffträger-Dispersionen sind in EP-B-0 605 497, EP-B-0 167 825 und US 5,885,486 beschrieben. Insbesondere für geeignete stabilisierende Substanzen und Ladungsstabilisatoren wird auf EP-B-0 605 497 verwiesen.

Die Erfindung wird durch die nachstehenden Beispiele näher erläutert, die eine Beanspruchte Ausführungsform (Erfindung) und nicht beanspruchte Beispiele (Vorgleich) zeigen.

### Beispiele

### a) P/O für P/O/W-Waschemulsion (Vergleich)

| **Handelsname** | | | **CTFA/INCI** | **PO-1-1/0** | **PO-2-1/0** |
|---|---|---|---|---|---|
| | | | | **[Gew.-%]** | **[Gew.-%]** |
| **Phase A** | | | | bt | bt |
| Dow Corning Formulation Aid | DC | 5225 | Cyclomethicone, dimethicone copolyol | 40,00 | 40,00 |

| **Phase B** | | | | | |
|---|---|---|---|---|---|
| Propylenglycol | | | Propylene glycol | 48,00 | 48,00 |
| FRAG 261346 "sunrise" | | | Fragrance | 12,00 | 0,00 |
| Chlorealis | | | Fragrance (Drogoco) | 0,00 | 12,00 |
| **total:** | | | | **100,00** | **100,00** |
| pH-Wert | | | | | |

Die Herstellung erfolgt durch Eintragen der Phase B in Phase A bei Raumtemperatur. Es wird für zwei Minuten homogenisiert.

### b) P/O/W-Waschemulsion (Vergleich)

| | | | **POW-1-1/0** | **POW-2-1/0** |
|---|---|---|---|---|
| **Handelsname** | | **CTFA/INCI** | **[Gew.-%]** | **[Gew.-%]** |
| | **Phase A** | | bt | bt |
| Texapon NSO | | Sodium lauryl ether (2) sulfate | 10,00 | 10,00 |
| Keltrol | | Xanthan grum | 0,50 | 0,50 |
| demin. Wasser | | Aqua | 84,50 | 84,50 |

| | **Phase B** | | | |
|---|---|---|---|---|
| PO-1-1/0 | | | 5,00 | 0,00 |
| PO-2-1/0 | | | 0,00 | 5,00 |
| **total:** | | | **100,00** | **100,00** |

Die Herstellung erfolgt durch Eintragen von Phase B in A und nachfolgendes Rühren mit einem Spatel.

Die Stabilität der POW-Waschemulsion beträgt mindestens 2 Monate bei 40 °C. Die Emulsion überlebt 5 Tau-Gefrier-Zyklen.

### c) Solid Lipid Nanoparticle mit verkapseltem Duftöl (Erfindung)

| **Handelsname** | | **Hersteller** | **CTFA/INCI** | **SLN-2-2/0** |
|---|---|---|---|---|
| | | | | **[Gew.-%]** |
| | **Phase A** | | | |
| demin. Water | | - | Aqua | 15,00 |
| Keltrol | | Kelco | Xanthan gum | 0,40 |

| | **Phase B** | | | |
|---|---|---|---|---|
| Cetiol MM Pastillen | | Cognis | Myristyl myristate | 20,0 |
| Fragrance Design 72515J | | Shaw Mudge | Fragrance | 10,0 |
| Pationic 138 A | | RITA | Sodium lauroyl lactylate | 0,50 |
| Vitamin E | | | d,I-a-Tocopherol | 1,00 |
| Ceralution H | | Sasol | Behenyl alcohol, glyceryl stearate, glyc | 1,25 |
| Vitamin C Dipalmitat | | Nikkol | Ascorbyl dipalmitate | 1,00 |

| | **Phase C** | | | |
|---|---|---|---|---|
| demin. Water | | - | Aqua | 50,2 |
| Phase D | | | | |
| Phenonip | | Nipa | Phenoxyethanol, methylparaben, ethyl | 0,60 |
| | **total:** | | | **100,0** |
| **Teilchengrößenverteilung nach der Herstellung** | | | | |
| Median [µm] | | | | 0,42 |
| > 1 µm [%] | | | | 100 |
| cm²/cm³ | | | | 140670 |

Die Herstellung erfolgt durch Aufheizen der Phasen B (ohne Duftstoffe) und Phase A auf 70 °C. Sodann wird der Duftstoff in Phase B eingetragen, Phase B wird langsam zu Phase A gegeben, und es wird homogenisiert Sodann wird die Phase AB mit Phase C bei 60 °C verdünnt und nochmals homogenisiert. Abschließend wird Phase D zugegeben.

Die Stabilität der SLN-Dispersion beträgt mehr als 7 Wochen bei 40 °C. Die Emulsion überlebt mehr als 5 Tau-Gefrier-Zyklen.

### d) Perlglanz -Waschgels mit verkapseltem Duftöl (Vergleich)

| **Handelsname** | **Hersteller** | **CTFA/INCI** | **PG-3-1/0** | **PG-4-1/0** | **PG-5-1/0** |
|---|---|---|---|---|---|
| | | | **[Gew.-%]** | **[Gew.-%]** | **[Gew.-%]** |
| demin. Water | - | Aqua | 44,00 | 30,30 | 33,50 |
| Ceralution F | Sasol | Sodium lauroyl lactylate, sodium dicocoy | 10,00 | 0,00 | 0,00 |
| Pationic 138 A | RITA | Sodium lauroyl lactylate | 0,00 | 5,00 | 5,00 |
| Protelan AGL 95 | Zschimmerer & Sc | Sodium lauroyl glutamate | 0,00 | 0,00 | 13,50 |
| Protelan LS 9011 1 | Zschimmerer &Sc | Natriumlauroylsarcosinat | 0,00 | 16,70 | 0,00 |
| Tegin D 1102 | goldschmidt | PEG-3 stearate | 10,00 | 10,00 | 10,00 |
| Atlas G 4829 | Uniqema | Laureth-9 | 18,00 | 18,00 | 18,00 |
| Glycerin lactate | Sasol | Glycerin lactate | 8,00 | 8,00 | 8,00 |
| Fragrance Design 72515J | Shaw Mudge | Fragrance | 10,00 | 10,00 | 10,00 |
| Vitamin C Dipalmitat | Jan Dekker | Ascorbyl dipalmitate | 0,00 | 1,00 | 1,00 |
| Vitamin E | Roche | d,I-a-Tocopherol | 0,00 | 1,00 | 1,00 |
| **total** | | | **100,0** | **100,0** | **100,0** |

Die Herstellung erfolgt durch Aufschmelzen der Inhaltstoffe bei Temperaturen von 60 bis 70 °C. Nach Vermischen der aufgeschmolzenen Inhaltsstoffe wird das Duftöl eingetragen, und es wird auf Raumtemperatur unter Rühren abgefühlt.

Die Perlglanz-Waschgele wiesen eine Stabilität von mehr als 7 Wochen bei 40 °C auf. Die Waschgele überlebten mehr als 5 Tau-Gefrier-Zyklen.

### e) Waschgel mit Perlglanz (Vergleich)

| **Handelsname** | | **Hersteller** | **CTFA/INCI** | **REGEL [Gew.-%]** | **PG-3-2/0** | **PG-5-2/0** | **PG-6-2/0** |
|---|---|---|---|---|---|---|---|
| | | | | | **[Gew.-%]** | **[Gew.-%]** | **[Gew.-%]** |
| | **Phase A** | | | | | | |
| Texapon NSO | | Cognis | Sodium lauryl ether (2) sulfate | 30,00 | 30,00 | 30,00 | 30,00 |
| Fragrance | | Shaw | Fragrance | 1,00 | 0,00 | 0,00 | 0,00 |
| Design 72515J | | Mudge | | | | | |
| demin. Water | | | | 65,50 | 56,50 | 56,50 | 56,50 |
| NaCl | | Merck | sodium chloride | 3,50 | 3,50 | 3,50 | 3,50 |

| | **Phase C** | | | | | | |
|---|---|---|---|---|---|---|---|
| PG-3-1/0 | | Shaw Mudge | Fragrance | 0,00 | 10,00 | 0,00 | 0,00 |
| PG-4-1/0 | | | | 0,00 | 0,00 | 10,00 | 0,00 |
| PG-5-1/0 | | | | 0,00 | 0,00 | 0,00 | 10,00 |
| | **total:** | | | **100,00** | **100,00** | **100,00** | **100,00** |

Die Herstellung erfolgt durch Verdünnen der Inhaltsstoffe von Phase A bei Raumtemperatur, Zugeben von Phase B und moderates Homogenisieren.

Von den Zusammensetzungen wurden Headspace-Chromatogramme erstellt, die die Entwicklung von Duftstoffkomponenten über einen Zeitraum in der Gasphase verfolgen.

Die Waschgele aus Beispiel e) wurden auf die menschliche Haut aufgetragen. Dabei wurde zunächst direkt nach dem Auftragen ein Headspace-Chromatogramm (Gaschromatogramm) erstellt Es ergaben sich für alle Gele relativ ähnliche Signale. Sämtliche Inhaltsstoffe der Duftölzusammensetzung traten auf.

Nach dem Abwaschen des Waschgels von der Haut ergaben sich wesentlich kleinere Signale im Headspace-Chromatogramm. Für die Waschgele WG-320, WG-520 und WG-620 ergaben sich stärkere Signale als für das Referenz-Waschgel. Dies deutet auf eine größere auf der Haut verbliebene Menge des Duftöls hin.

Nach einer Wartezeit von einer Stunde nach dem Abwaschen wurde wiederum ein Headspace-Chromatogramm für alle Hautoberflächen, die mit den unterschiedlichen Waschgelen behandelt waren, aufgenommen. Es ergaben sich für die Waschgele von Beispiel e) wesentlich stärkere Signale, insbesondere für Hexylacetat und Limonan, im Vergleich zum Referenzgel. Auch im Bereich von Phenoxiethylisobutyrat ergaben sich deutlich stärkere Signale für die Waschgele von Beispiel e). Hierdurch wird deutlich, dass die Waschgele bzw. die darin enthaltenen Duftöle besser auf die Haut aufziehen und eine zeitverzögerte Freisetzung bewirken. Hierdurch dauert das Dufterlebnis beim Einsatz der Waschgele wesentlich länger an als bei Verwendung des Referenzwaschgels.

## Patentansprüche

1. Duftstoff- und/oder Aromen-Zusammensetzung zur gezielten Freisetzung von Duftstoffen und/oder Aromen in Form einer Solid-Lipid-Nanoparticle (SLN)-Dispersion, in der Nanopartikel auf Lipidbasis vorliegen, die durch eine Emulgatorenmonoschicht, eine oder mehrere Membranschichten oder andere Hilfsmittel stabilisiert sind, wobei in den Nanopartikeln und/oder in der Emulgatormonoschicht oder den Membranschichten die Duftstoffe und/oder Aromen eingeschlossen sind, herstellbar durch
a) Vermischen des Duftstoffs und/oder Aromas mit dem Wirkstoffträger auf Lipidbasis und mindestens einem Emulgator, der in Stufe b) zur Ausbildung einer lyotropen flüssigkristallinen Mischphase führt, bei einer Temperatur oberhalb des Schmelz- oder Erweichungspunktes des Wirkstoffträgers, zur Ausbildung einer Phase B,
b) mechanisches Vermischen der Phase B mit einer wässrigen Phase oder Polyolphase A, die einen Emulgator enthalten kann, bei einer Temperatur oberhalb des Schmelz- oder Erweichungspunktes des Wirkstoffträgers, wobei das Gewichtsverhältnis von Phase B zu Phase A1 : 5 bis 5 : 1 beträgt, ohne Hochdruckhomogenisierung, zur Ausbildung einer lyotropen flüssigkristallinen Mischphase,
c) Verdünnen der Mischphase mit einer wässrigen Phase oder Polyolphase, die einen Emulgator enthalten kann, bei einer Temperatur der wässrigen Phase oder Polyolphase, die unter dem Schmelz- oder Erweichungspunkt des Wirkstoffträgers liegt, unter Rühren und ohne Hochdruckhomogenisierung, auf eine gewünschte Endkonzentration der Dispersion, wobei in der Duftstoff- und/oder Aromen-Zusammensetzung Lipide und Emulgatoren in einem Gewichtsverhältnis von 50 : 1 bis 2 : 1 vorliegen.

2. Zusammensetzung nach Anspruch 1, **dadurch gekennzeichnet, dass** Nanopartikel auf Lipidbasis vorliegen, die Emulgatoren oder Tenside mit hydrophilen und hydrophoben Resten enthalten, wobei im Bereich der hydrophoben Reste der Emulgatoren oder Tenside Duftstoffe und/oder Aromen eingebracht sind.

3. Zusammensetzung nach Anspruch 1 oder 2, in der 0,1 bis 30 Gew.-% Teilchen auf Lipidbasis und 0,1 bis 30 Gew.-% Duftstoffe und/oder Aromen vorliegen, bezogen auf das Gesamtgewicht der Zusammensetzung.

4. Zusammensetzung nach einem der Ansprüche 1 bis 3, die Teilchen auf Lipidbasis mit unterschiedlichen Schmelrpunkten enthält.

5. Zusammensetzung zur gezielten Freisetzung des Duftstoffs und/oder Aromas nach einem der Ansprüche 1 bis 4 in Form einer kosmetischen, pharmazeutischen, lebensmitteltechnologischen oder waschmitteltechnologischen Zusammensetzung.

## Claims

1. A fragrance and/or aroma substance composition for the targeted release of fragrances and/or aroma substances in the form of a solid lipid nanoparticle (SLN) dispersion where there are lipid-based nanoparticles that are stabilised by a monolayer of an emulsifying agent, one or several membrane layers or other auxiliary agents, the fragrance and/or aroma substances being enclosed in the nanoparticles and/or in the emulsifying agent monolayer or the membrane layers, manufacturable by
a) mixing of the fragrance and/or aroma substance with the lipid-based active agent carrier and at least one emulsifying agent that in step b) leads to the formation of a lyotropic liquid crystalline mixed phase, at a temperature above the melting or softening point of the active substance carrier to form a phase B,
b) mechanical mixing of the phase B with an aqueous phase or polyol phase A that can contain an emulsifying agent at a temperature above the melting or softening point of the active substance carrier, the weight ratio of phase B to phase A being 1 : 5 to 5 : 1, without high pressure homogenisation, to form a lyotropic liquid crystalline mixed phase,
c) dilution of the mixed phase with an aqueous phase or polyol phase that can contain an emulsifying agent, at a temperature of the aqueous phase or polyol phase below the melting or softening point of the active substance carrier while agitating and without high pressure homogenisation to a desired final concentration of the dispersion, lipids and emulsifying agents in a weight ratio of 50 : 1 to 2 : 1 being present in the fragrance and/or aroma substance composition.

2. The composition according to Claim 1, **characterised in that** there are lipid-based nanoparticles that contain emulsifying agents or surfactants with hydrophilic or hydrophobic residues, fragrances and/or aroma substances being introduced in the region of the hydrophobic residues of the emulsifying agents or surfactants.

3. The composition according to Claim 1 or 2, in which there are 0.1 to 30 % by weight lipid-based particles and 0.1 to 30 % by weight fragrances and/or aroma substances based on the total weight of the composition.

4. The composition according to any of Claims 1 to 3 that contains lipid-based particles with different melting points.

5. A composition for the targeted release of the fragrance and/or aroma substance according to any of Claims 1 to 4 in the form of a cosmetic, pharmaceutical, food technology or detergent technology composition.

## Revendications

1. Composition de parfum et/ou d'arômes destinée à une libération ciblée de parfums et/ou d'arômes sous la forme d'une dispersion de nanoparticules lipidiques solides (SLN), dans laquelle les nanoparticules sont à base de lipides qui sont stabilisés par une monocouche d'émulsifiants, une ou plusieurs couches de membrane, ou d'autres excipients, dans laquelle les parfums et/ou les arômes sont enfermés dans les nanoparticules et/ou dans la monocouche d'émulsifiants ou dans les couches de membrane, qui peut être fabriquée par :
a) mélange du parfum et/ou de l'arôme avec le vecteur de principe actif à base lipidique et au moins un émulsifiant, qui mène à l'étape b) à la formation d'une phase mixte de cristaux liquides lyotropes, à une température supérieure au point de fusion ou au point de ramollissement du vecteur de principe actif, afin de former une phase B,
b) mélange mécanique de la phase B avec une phase aqueuse ou une phase polyol A, qui peut contenir un émulsifiant, à une température supérieure au point de fusion ou au point de ramollissement du vecteur de principe actif, où le rapport de masse entre phase B et phase A est de 1 :5 jusqu'à 5 :1, sans homogénéisation sous haute pression, pour former une phase mixte à cristaux liquides lyotropes,
c) dilution de la phase mixte avec une phase aqueuse ou une phase polyol, qui peut contenir un émulsifiant, à une température de la phase aqueuse ou de la phase polyol qui est inférieure au point de fusion ou au point de ramollissement du vecteur de principe actif, sous agitation et sans homogénéisation sous haute pression, pour obtenir une concentration finale souhaitée de la dispersion, dans laquelle dans la composition de parfum et/ou d'arômes les lipides et les émulsifiants sont utilisés selon un rapport de masse de 50:1 jusqu'à 2:1.

2. Composition selon la revendication 1, **caractérisée en ce que** sont présentes des nanoparticules à base de lipides qui contiennent des émulsifiants ou des tensioactifs ayant des radicaux hydrophiles et hydrophobes, où les parfums et/ou arômes sont introduits dans la zone des restes hydrophobes des émulsifiants ou des tensioactifs.

3. Composition selon la revendication 1 ou 2, dans laquelle sont présentes 0,1 à 30 % en masse de particules à base lipidique et 0,1 à 30 % en masse de parfums et/ou d'arômes, sur la base de la masse totale de la composition.

4. Composition selon l'une des revendications 1 à 3, qui contient des particules à base de lipides ayant des points de fusion différents.

5. Composition destinée à une libération ciblée du parfum et/ou de l'arôme selon l'une des revendications 1 à 4 sous la forme d'une composition cosmétique, pharmaceutique, des technologies des denrées alimentaires ou des technologies des détergents.
